# EUROPEAN PATENT SPECIFICATION

(11) **EP 2 774 616 B1**
(45) Date of publication and mention of the grant of the patent: **29.04.2020**
(21) Application number: 11875013.2
(22) Date of filing: 22.12.2011
(51) Int. Cl.: A61K 35/74, A61P 3/04, A61P 3/10, C12R 1/01

(54) **APPLICATION OF ROSEBURIA IN TREATING AND PREVENTING OBESITY RELATED DISEASES**
VERWENDUNG VON ROSEBURIA BEI DER BEHANDLUNG UND VORBEUGUNG ERKRANKUNGEN IM ZUSAMMENHANG MIT ADIPOSITAS
APPLICATION DE ROSEBURIA DANS LE TRAITEMENT ET LA PRÉVENTION DE MALADIES LIÉES À L'OBÉSITÉ

(30) Priority: 02.11.2011 CN 201110342339
(43) Date of publication of application: 10.09.2014
(73) Proprietor: BGI Shenzhen, Shenzhen, Guangdong 518083 (CN); BGI Shenzhen Co., Limited, Shenzhen, Guangdong 518083 (CN)
(72) Inventor: QIN, Junjie, Shenzhen Guangdong 518083 (CN); LI, Shenghui, Shenzhen Guangdong 518083 (CN); ZHU, Jianfeng, Shenzhen Guangdong 518083 (CN); LI, Xiaoping, Shenzhen Guangdong 518083 (CN); LIANG, Suisha, Shenzhen Guangdong 518083 (CN); WANG, Jun, Shenzhen Guangdong 518083 (CN); WANG, Jian, Shenzhen Guangdong 518083 (CN); YANG, Huanming, Shenzhen Guangdong 518083 (CN); FENG, Qiang., Shenzhen Guangdong 518083 (CN)
(74) Representative: Huenges, Martin
(86) International application number: PCT/CN2011/084450
(87) International publication number: WO 2013/063849

(56) References cited:
- WO-A2-2008/076696
- CN-A- 101 580 550
- SYLVIA H DUNCAN ET AL: "Proposal of Roseburia faecis sp. nov., Roseburia hominis sp. nov. and Roseburia inulinivorans sp. nov., based on isolates from human faeces", INTERNATIONAL JOURNAL OF SYSTEMATIC AND EVOLUTIONARY MICROBIOLOGY, SOCIETY FOR GENERAL MICROBIOLOGY, READING, GB, vol. 56, no. PT 10, 1 October 2006 (2006-10-01), pages 2437-2441, XP002692349, ISSN: 1466-5026, DOI: 10.1099/IJS.0.64098-0
- DUNCAN S H ET AL: "ROSEBURIA INTESTINALIS SP. NOV., A NOVEL SACCHAROLYTIC, BUTYRATE-PRODUCING BACTERIUM FROM HUMAN FAECES", INTERNATIONAL JOURNAL OF SYSTEMATIC AND EVOLUTIONARY MICROBIOLOGY, SOCIETY FOR GENERAL MICROBIOLOGY, READING, GB, vol. 52, no. 5, 1 January 2002 (2002-01-01), pages 1615-1620, XP009035387, ISSN: 1466-5026, DOI: 10.1099/IJS.0.02143-0
- AUDREY M. NEYRINCK ET AL: "Prebiotic Effects of Wheat Arabinoxylan Related to the Increase in Bifidobacteria, Roseburia and Bacteroides/Prevotella in Diet-Induced Obese Mice", PLOS ONE, vol. 6, no. 6, 9 June 2011 (2011-06-09), pages e20944/1-12, XP55170799, US ISSN: 1932-6203, DOI: 10.1371/journal.pone.0020944
- LI M.: 'Co-variation analysis of human gut microbial structure and host global metabolism' THESIS FOR PHILOSOPHY DEGREE 23 February 2010, pages 1 - 149, XP008173177

## Description

### FIELD

The present disclosure relates to the field of microbiology, specifically, relates to *Roseburia* bacterial strains for use in the treatment and prevention of obesity-related diseases, and also involves the composition comprising *Roseburia* bacteria and the application thereof.

### BACKGROUND

Obesity is a chronic disease, and many factors can lead to obesity. The incidence of origin is not clear so far. Obesity is also an inducing factor for a series of diseases, such as hypertension, diabetes, coronary heart disease, gallbladder disease, osteoarthritis, sleep apnea, respiratory disorders, uterus tumor, prostate cancer, breast cancer and colon cancer. According to NIH report, about 97 million Americans are overweight and suffer from obesity, including about 15.1 million people suffering from obesity associated with type II diabetes. Each year nearly 200 thousand people die from obesity-related diseases.

Obesity is usually caused by changes in physiological or biochemical function which lead to excess body fat. Fat generally includes neutral lipid, phospholipid and cholesterol. Fat increase is due to that energy intake is greater than energy consumption. According to pathogenesis, there are two types of obesity: (a) simple obesity and (b) second obesity. Simple obesity can be divided into idiopamic obesity and acquired obesity. The number of simple obesity patients may be more than 95% of the total number of obesity. Idiopamic obesity is caused by a large number of fat cells, and is common in childhood obesity. Acquired obesity is due to larger fat cells, and is common in adult obesity. Second obesity is also known as symptomatic obesity, which is usually caused by endocrine or metabolic diseases.

There are five treatment strategies for obesity: diet, exercise, behavior therapy, drug treatment and therapeutic operation. Which strategy to be chosen depends on patients' health risk factors and the speed and effectiveness of weight loss. Combination of these strategies can also be used for the treatment of obese patients. The speed and effectiveness of their weight loss are influenced by many factors such as age, height, family history and risk factors. Diet-exercise therapy is to eat low-calorie, low-fat foods combined with aerobic exercise, and requires long-term adherence to be effective. Thus, this strategy is generally considered to be not successful to the general public. Surgical removal of body fat can achieve immediate results, but there are many restrictions, such as surgical risk, effects which are difficult to sustain and too much expensive cost.

Drug therapy is the main clinical treatment of obesity and obesity-related diseases (such as diabetes) method. Mechanism of drug therapy includes suppressing appetite, increasing energy consumption, stimulating fat movement, reducing triglyceride synthesis and inhibiting fat absorption. The main drugs at present are phenylpropanolamine (PPA), orlistat (Xenical III) and sibutramine (ReductilTM). The high blood glucose of some diabetes patients still cannot be properly controlled through diet and/or exercise therapy or the use of such therapeutic compounds. For these patients, exogenous insulin should be used. To these patients, the use of exogenous insulin is very expensive and a painful way, which causes multiple complications. For example, the absence of meals or abnormal exercise, and insulin-dose miscalculation can lead to insulin response (low blood sugar). In addition, the use of drugs may also occur local or systemic drug allergies or immune resistance.

WO 2013/050792 describes the use of *Roseburia hominis* to regulate appetite in a subject.

Devillard et al. (J Bacteriol, 2007, 189(6):2566-2570) investigate the metabolism of linoleic acid by human gut bacteria.

Mirande et al. (J Appl Microbiol, 2010, 109:451-460) investigate the dietary fibre degradation and fermentation by two bacteria from the human intestine.

At present, there is still no method or drug which is effective with fewer side effects for treatment and prevention of obesity and its related diseases. So there is an urgent need to develop a new, non-toxic drug for the treatment and prevention of obesity and its related diseases in this field.

### SUMMARY

The invention is described in the claims.

One aim of the present disclosure is to provide *Roseburia* for use in the prevention and treatment of obesity related diseases.

Another aim of the present disclosure is to provide pharmaceutical, drink, food composition, or animal feed composition for use in the prevention and treatment of obesity related diseases.

The third aim of the present disclosure is to provide *Roseburia,* a pharmaceutical, drink or food composition for use in a method of reducing body weight and/or blood glucose and application thereof.

According to a first aspect of the present disclosure, there is provided *Roseburia* in the preparation of the composition for use in the treatment and/or prevention of obesity.

In one preferred embodiment, the composition is selected from the group consisting of pharmaceutical composition, food composition, drink composition, or feed composition.

In another preferred embodiment, the *Roseburia* is *Roseburia inulinivorans.*

In another preferred embodiment, the *Roseburia* is *Roseburia inulinivorans* DSM 16841.

In another preferred embodiment, the composition is for use in improving the tolerance of blood glucose.

According to a second aspect of the present disclosure, there is provided a food composition, the food composition comprises an effective amount of *Roseburia* and a carrier acceptable for food composition, wherein *Roseburia* is not *Roseburia hominis.*

In another preferred embodiment, the forms of the food composition are selected from solid emulsion, solution agent, powder agent, or suspension agent.

In another preferred embodiment, the food composition is for use in reducing blood glucose and/or body weight, and/or used in improving the tolerance of blood glucose.

In another preferred embodiment, the effective amount of *Roseburia* is 1×10-1×10²⁰ cfu/mL(or cfu/g), preferably 1×10⁴-1×10¹⁵cfu/mL(or cfu/g).

According to a third aspect of the present disclosure, there is provided a pharmaceutical composition, the pharmaceutical composition comprises an effective amount of *Roseburia,* and the pharmaceutically acceptable carrier, wherein *Roseburia* is not *Roseburia hominis.*

In another preferred embodiment, the forms of the pharmaceutical composition are selected from granule, capsule, tablet, powder agent, oral liquid, suspension, and/or emulsion.

In another preferred embodiment, the pharmaceutical composition is for use in reducing blood glucose and/or body weight, and/or used in improving the tolerance ability of blood glucose.

In another preferred embodiment, the effective amount of Roseburia is 1×10-1×10²⁰ cfu/mL(or cfu/g), preferably 1×10⁴-1×10¹⁵cfu/mL(or cfu/g).

In another preferred embodiment, a recipe of the food composition according to the second aspect or the pharmaceutical composition according to the third aspect comprises: 1×10-1×10²⁰ cfu/mL of *Roseburia,* and a pharmaceutically acceptable carrier or a carrier acceptable for food compositions, and/or an excipient.

In another preferred embodiment, a recipe of the food composition according to the second aspect or the composition according to the third aspect comprises: 1×10⁴-1×10¹⁵cfu/mL of *Roseburia,* and a pharmaceutically acceptable carrier or a carrier acceptable for food compositions, and/or an excipient.

In another preferred embodiment, a recipe of the food composition according to the second aspect or the pharmaceutical composition according to the third aspect comprises: 1×10⁶-1×10¹¹ cfu/mL of *Roseburia,* and a pharmaceutically acceptable carrier or a carrier acceptable for food compositions, and/or an excipient.

According a fourth aspect of the present disclosure, there is provided the food composition according to the second aspect or the pharmaceutical composition according to the third aspect, the composition is for use in reducing the blood glucose and/or body weight of subjects.

In another preferred embodiment, the composition is for use in improving the tolerance of blood glucose.

According a fifth aspect of the present disclosure, there is provided a *Roseburia* according to the first aspect of the present disclosure, the food composition according to the second aspect, and/or the pharmaceutical composition according to the third aspect for use in a method of reducing body weight and/or blood glucose comprising feeding or applying a *Roseburia* according to the first aspect of the present disclosure, the food composition according to the second aspect, and/or the pharmaceutical composition according to the third aspect to subjects in need.

According a sixth aspect of the present disclosure, there is provided *Roseburia* for use in improving the tolerance of blood glucose.

According a seventh aspect of the present disclosure, there is provided a *Roseburia* according to the first aspect of the present disclosure, the food composition according to the second aspect, and/or the pharmaceutical composition according to the third aspect for use in a method of improving the tolerance of blood glucose, comprising administering a *Roseburia* according to the first aspect of the present disclosure, the food composition according to the second aspect, and/or the pharmaceutical composition according to the third aspect to subjects in need.

According an eighth aspect of the present disclosure, there is provided the *Roseburia* according to the first aspect of the present disclosure, the food composition according to the second aspect, and/or the pharmaceutical composition according to the third aspect for use in a method of prevention or treatment of obesity related diseases, comprising administering the *Roseburia* according to the first aspect of the present disclosure, the food composition according to the second aspect, and/or the pharmaceutical composition according to the third aspect to subjects in need.

In another preferred embodiment, the obesity related diseases are selected from the group consisting of diabetes, hypertension, hyperglycemia, hyperlipidemia, coronary heart disease, atherosclerosis, stroke, or the combination thereof.

According to all aspects of the present disclosure, the subjects is preferable mammals, more preferably mice or humans.

### BRIEF DESCRIPTION OF THE DRAWINGS

Figure 1 illustrates the grouping information and steps of the treatment in embodiments of the present disclosure;
Figure 2 illustrates the increase tendency of the body weight of three groups of mice within 10 weeks after feeding;
Figure 3 illustrates the differences of the significances (P values) in the increase of the body weight among three groups of mice;
Figure 4 illustrates the changing curve of the blood glucose in the mice at 10th week after feeding;
Figure 5 illustrates the tolerance of blood glucose in treated mice at the 10th week after feeding.

### DETAILED DESCRIPTION

With extensive experiments and in-depth research, it is surprisingly discovered the use of *Roseburia* in prevention and treatment of obesity-related diseases. After applying the active composition comprising *Roseburia* to experimental subjects, it is surprisingly discovered that the composition is capable of inhibiting body weight growth, reducing blood glucose, improving the tolerance ability of blood glucose, ameliorating diabetes and obesity effectively. The present disclosure is based on these discoveries.

As used herein, the term "comprise" or "comprising" indicates all components can be used in the mixture or composition in the present disclosure. So the meaning of "mainly composed of', "consist mainly of', and "consisted of' belongs to the scope of the term "comprise" or "comprising".

As used herein, the term *"Roseburia* bacterial strain" and *"Roseburia"* can be used interchangeably. In a preferred embodiment, the bacterial strain is *Roseburia inulinivorans* DSM 16841, derived from DSMZ (Deutsche Sammlung von Mikroorganismen und Zellkulturen GmbH). The physiological properties of *Roseburia* are as follows: slightly-curved-rod shape, about 0.5µm×2.3∼5.0µm in size, without spores, motile, having a cluster of flagella at a proximal end or one end of the concave of a cell. It may ferment several carbohydrates including wood sugar, galactose, raffinose, glucose, maltose, cellobiose, sucrose, starch and glycogen, mainly producing or only producing butyric acid. The cultivation conditions are: anaerobic cultured in M2GSC media at 37°C for 2-5 days.

The present disclosure discloses *Roseburia* for use in the prevention and treatment of obesity and its related diseases. The bacterial strain DSM 16841 has the property of inhibiting the growth of the body weights of subjects after intaking high-fat food. According to one embodiment of the present disclosure, the CF57BL/6J mice that are fed with high-fat food which will induce obesity are treated with the bacterial strain DSM 16841. Comparing with the untreated control group, the body weight of the bacterial treated group retain unchanged which is beneficial for reducing blood glucose level. Therefore, the bacterial strain can be applied to prevent and treat obesity and its related diseases, e.g., diabetes. etc.

The present disclosure further provides a composition, preferably a pharmaceutical composition. The composition comprises an effective amount of the *Roseburia* bacterial strain, wherein *Roseburia* is not *Roseburia hominis.* In one preferable embodiment, the composition further comprises the bacterial strains selected from the group consisting of: *Lactobacillus gasseri, Lactobacillus rhamnosus* GM-020, *Lactobacillus rhamnosus* PL60 or the combination thereof.

The pharmaceutical compositions can be administrated in forms of tablet, capsule or injection. These pharmaceutic compositions comprise excipients, pharmaceutically acceptable media and carriers. Appropriate excipients, media and carriers may be selected depending on the routes of administration. The pharmaceutical compositions in the present disclosure can further comprise auxiliary active constituents.

The carrier, excipient or thinner comprises but is not limited to lactose, glucose, sucrose, sorbitol, mannose, starch, gum arabic, calcium phosphate, alginate, gelatin, calcium silicate, fine crystalline cellulose, polyvinyl pyrrolidone (PVP), cellulose, water, syrup, methyl cellulose, methyl p-hydroxybenzoate, propyl para-hydroxybenzoate esters, talc, magnesium stearate or mineral oil, *etc.*

The pharmaceutical composition can further comprise lubricant, wetting agent, emulsifier, suspension stabilizer, preservative, sweetener, spices, *etc.* The pharmaceutical composition in the present disclosure can be manufactured as enteric coatings by several known method in the art, so that the active constituent of the pharmaceutical composition, i.e., microorganism, can be protected from gastric acid and go through the stomach intactly.

Furthermore, microorganisms of the present disclosure may be administered in a form of capsule prepared by conventional process. For example, standard excipient and lyophilized microorganisms of the present disclosure are mixed together and prepared to pellets and then, the pellets are filled into gelatin capsules. In addition, the microorganisms of the present disclosure and pharmaceutically allowable excipient, for example, aqueous gum, cellulose, silicates or oi1, are mixed to produce a suspension or emulsion and then, the suspension or emulsion can be filled into soft gelatin capsule.

The pharmaceutical composition of the present disclosure may be prepared as enterically coated tablets for oral administration. The term "the enteric coating" of the present disclosure includes all conventional pharmaceutically acceptable coating that has resistance to gastric juice, but can disintegrate sufficiently in small intestine for a rapid release of the microorganisms of the present disclosure. The enteric coating of the present disclosure can be maintained for more than 2 hours in synthetic gastric juice, such as HCl solution having a pH of about 1 at the temperature of 36-38°C, and preferably decomposes within 1.0 hours in synthetic intestinal juice, such as buffer solution of pH 7.0.

The enteric coating of the present disclosure applies to each tablet with the amount of about 16 to 30 mg, preferably 16 to 25 mg, more preferably 16 to 20 mg. The thickness of enteric coating of the present disclosure is 5 to 100µm, preferably 20 to 80µm. The components of the enteric coating are selected appropriately from commonly known polymeric materials.

The preferred enteric coating of the present disclosure are prepared from polymers of cellulose acetate phthalate or polymers of trimellitates and methacrylate copolymer (for example, methacrylic acid copolymer contains more than 40% of methacrylic acid and contains methyl-cellulose-phthalate-hydroxypropyl methacrylate or derivatives thereof).

The cellulose acetate phthalate employed in the enteric coating of the present disclosure has a viscosity of about 45 cP to 90 cP, which contains 17% to 26% of acetyl, and 30% to 40% of phthalate. The cellulose acetate trimelitate used in the enteric coating has a viscosity of about 15 cs to 21cs, which contains 17% to 26% of acetvl. The cellulose acetate trimelitate manufactured by the Eastman Kodak Company may be used as a material for the enteric coating of the present disclosure.

Hydroxyprophyl methylcellulose phthalate used in the enteric coating of the present disclosure has a molecular weight of generally 20,000 to 130,000 dalton, preferably 80,000 to 100,000 dalton which contains 5% to 10% hydroxyprophyl, 18% to 24% of metoxy contents, and 21 to 35% of phthalyl contents.

Hydroxyprophyl methyl cellulose phthalate used in the enteric coating of the present disclosure is HP50 manufactured by Shin-Etsu Chemical Co. Ltd., Japan. The HP50 contains 6% to 10% hydroxyprophyl, 20% to 24% metoxy, 21% to 27% prophyl, and has a molecular weight of 84,000 dalton. Another material for enteric coating is HP55, which contains 5% to 9% hydroxyprophyl, 18% to 22% metoxy, 27% to 35% phthalate, and has a molecular weight of 78,000 dalton.

The enteric coating of the present disclosure is prepared by using conventional methods of spraying the enteric coating solution to the core. Solvents used in the process of the enteric coating are alcohol such as ethanol, ketone such as acetone, halogenated hydrocarbon such as dichloromethane, or the mixture thereof. Softeners such as di-n-butylphthalate and triacetin are added to the enteric coating solution in the ratio of 1 part coating material to about 0.05 or to about 0.3 part softner. A spraying process is preferably performed continuously, and the amount of materials sprayed may be controlled depending on the condition of the coating process. Spraying pressure may be regulated variously and, generally, desirable result can be obtained under the pressure of average 1 to 1.5 bar.

As used herein, the term "pharmaceutically effective amount" refers to an amount of a compound sufficient to achieve desirable function or activity in humans and/or animals in need and acceptable to such humans and/or animals. In the present disclosure, the effective amount contains 1×10-1×10²⁰ cfu/mL *Roseburia* may be manufactured, preferably the effective amount contains 1×10⁴-1×10¹⁵cfu/mL *Roseburia* may be manufactured, further preferably the effective amount contains 1×10⁶-1×10¹¹ cfu/mL *Roseburia* may be manufactured.

The effective amount of *Roseburia* in the preparation of the pharmaceutical composition can vary depending on the mode of administration and the severity of the disease to be treated. The dosage form suitable for oral administration comprises 1×10-1×10²⁰cfu/mL (or cfu/g) active *Roseburia* or active component produced by fermentation (preferably 1×10⁴-1×10¹⁵cfu/mL, further preferably 1×10⁶-1×10¹¹cfu/mL), which can be mixed with solid or liquid pharmaceutically acceptable carrier. This dose regimen may be adjusted to provide an optimal therapeutic response. For example, due to the urgent requirement of treatment conditions, several divided doses can be given daily, or the dosage can be reduced proportionally.

The *Roseburia* can be administrated orally. The solid carrier comprises starch, lactose, dicalcium phosphate, microcrystalline cellulose, sucrose and white clay; and the liquid carrier comprises media, polyethylene glycol, non-ionic surfactant, and edible oils (such as corn oil, peanut oil, and sesame oil). As long as they are suitable for the characteristic of *Roseburia* and special administration mode to be needed. The adjuvants can be incorporated in the preparation of the pharmaceutical composition advantageously, which comprise: flavoring agents, pigments, preservatives and antioxidants such as vitamin E, vitamin C, BHT and BHA.

In term of preparation and administration, the preferred pharmaceutical composition is solid composition, further preferably in forms of tablet and capsule filled with solid or liquid. The oral administration is preferred.

The composition of the present disclosure may be administrated once or more per day on the subject. The unit of administration amount refers to a dosage which may be separated physically and suitable for administration to human or all other mammalian individuals. Each unit contains a pharmaceutically acceptable carrier and an effective amount of the microorganisms of the present disclosure. The administration amount may vary depending on the weight and the severity of obesity of the patient, supplemental active ingredients included and microorganisms used therein. In addition, if possible, it may be administered separately, and, if necessary, it can be administered continuously. Therefore, the dosage will not impose restrictions to the present disclosure. Further, the "compositions" in the present disclosure means not only pharmaceuticals, but also can be used as a function food and health supplement. In one preferred embodiment, the compositions include beverage, food, drug, animal feed, *etc.*

In one preferred embodiment of the present disclosure, there is provided a food composition comprising an effective amount of *Roseburia,* and carrier acceptable for balance food, for use in reducing blood sugar and/or body weight of subjects, wherein *Roseburia* is not *Roseburia hominis.* The formulation of the food composition is selected from solid, dairy food, solution product, powder product, or suspension product.

In one preferred embodiment, there is also provided a pharmaceutical composition comprising a pharmaceutically effective amount of *Roseburia,* and pharmaceutically acceptable carrier, for use in reducing blood sugar and/or body weight of subjects, wherein *Roseburia* is not *Roseburia hominis.* The formulation of the pharmaceutical composition is selected from granule, capsule, tablet, powder agent, oral liquid, suspension, or emulsion.

The recipe of composition comprises: 1×10-1×10²⁰cfu/mL *Roseburia,* and a carrier acceptable pharmaceutically or for food compositions, and/or an excipient.

In another preferred embodiment, the recipe of the composition comprises: 1×10⁴-1×10¹⁵cfu/mL *Roseburia,* and a carrier acceptable pharmaceutically or for food compositions, and/or an excipient.

In another most preferred embodiment, the recipe of the composition comprises: 1×10⁶-1×10¹¹cfu/mL *Roseburia,* and a carrier acceptable pharmaceutically or for food compositions, and/or an excipient.

The present disclosure further provides the pharmaceutical composition, food composition, drink composition, and/or a combination thereof for use in a method of reducing body weight and/or blood glucose, and/or improving the tolerance of blood glucose.

In another preferred embodiment, the method comprises: administering the pharmaceutical composition, food composition, drink composition, and/or a combination thereof. The subject is human.

In another preferred embodiment, the method comprises: administering the pharmaceutical composition, food composition, drink composition, and/or a combination thereof. The subject is animals, preferable murine or leporid.

The advantages of the present disclosure include:
(a) The *Roseburia* in the present disclosure has excellent characteristics of prevention for obesity and related diseases;
(b) The *Roseburia* in the present disclosure has characteristics of treating obesity and related diseases, reducing blood glucose, improving the tolerance ability of blood glucose.

The following exemplary embodiments further describe the present disclosure. Further, for the embodiments in which details of the experimental methods are not described, such methods are carried out according to conventional conditions such as those described in Sambrook et al., Molecular Cloning: A Laboratory Manual (New York: Cold Spring Harbor Laboratory Pres, 1989), or according to the conditions in Microorganism: Laboratory manual (by James Cappuccino and Natalie Sherman, pressed in Pearson Education), or according to the condition suggested by the manufacturers.

### EXAMPLE 1

### Materials

Mice: Fifteen C57BL/6J mice were 6 weeks old at the beginning of the experiment and purchased from Laboratory Animal Center of Southern Medical University, China. The mice were fed with a control diet and housed in groups of five per cage in same environment (12-h daylight cycle, lights off at 6 p.m.) with free access to food and water.

Bacterial strain: *Roseburia inulinivorans* DSM 16841 was purchased from DSMZ (Deutsche Sammlung von Mikroorganismen und Zellkulturen GmbH) .

High-fat diet(HF) containing 58% fat (soybean oil and lard, g/100 g of total diet), 25.6% sucrose, and 16.4% protein was purchased from Beijing HFK Bioscience co., LTD, China.

Control diet was purchased from Laboratory Animal Center of Southern Medical University, China.

### EXAMPLE 2

### Methods of Grouping and Treatment

All the mice were divided into three groups (n=5/group).

At the beginning of the experiment, 6 weeks old C57BL/6J mice (no need for germ-free) were gavaged with freshly cultured *R. inulinivorans* DSM 16841. Oral glucose tolerance tests (OGTT) were performed on 10h fasted mice for blood glucose levels tests.

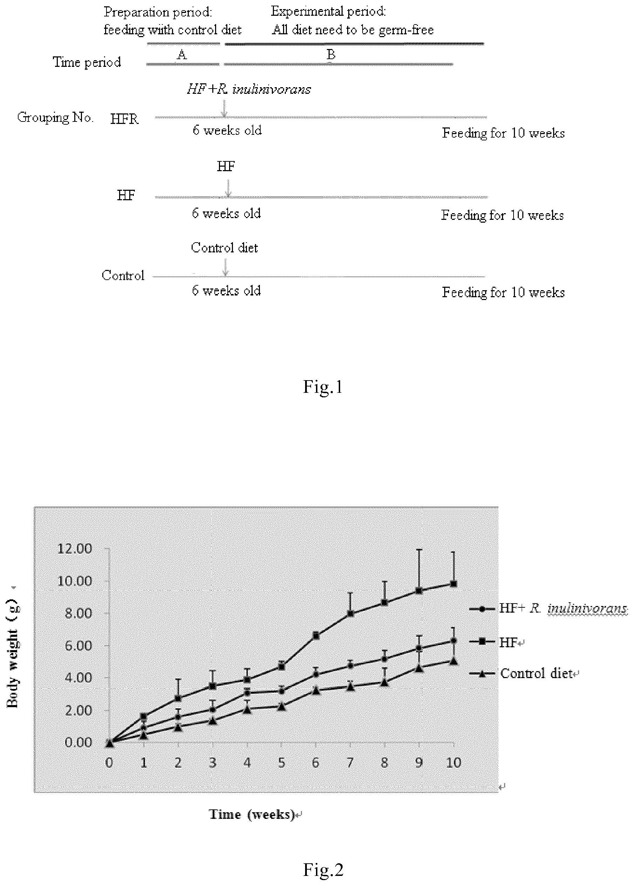

The detailed information and treatment methods were showed in Fig. 1.

After 3 weeks of acclimatization, these mice were housed in groups of five per cage in clean environment with free access to control food and water to reach 6 weeks old. Body weight of each mouse was monitored once a week while the food intake of each group was recorded every two days.

The 15 mice were divided into three groups (n=5/group). Their diets were modified as below. The control Group was fed with control diet, meanwhile the HF group and *Roseburia*-treated HFR group were fed with high-fat diet.

Each cage was maintained clean during the experimental period. All the mice lived in their corresponding cages and gavaged in unified processes.

*R. inulinivorans* DSM 16841 was freshly suspended in culture medium weekly with the concentration 1×10⁷cfu/mL, and the cultivation liquid compressed to 1×10⁸cfu/mL. Then the strain was orally administered to the mice by gavage (0.15mL/10g body weight) every two days.

Body weight and daily food intake of each mouse were recorded during the experiment. After 5 and 10 weeks of treatments, oral glucose tolerance tests (OGTT) were performed and blood glucose levels of 10h fasted mice were determined by using a glucose meter. Blood was collected from the tip of the tail vein both before and after glucose administration (60 min and 120 min).

### EXAMPLE 3

### Results

1. The average increases of body weight during the 10 weeks-treatment were shown in Table1 and Fig. 2.

**Table 1**

| Group | Time (week) | | | | | | | | | | |
|---|---|---|---|---|---|---|---|---|---|---|---|
| | 0 | 1 | 2 | 3 | 4 | 5 | 6 | 7 | 8 | 9 | 10 |
| 1.HFR(g) | 0.00 | 0.92 | 1.60 | 2.06 | 3.10 | 3.20 | 4.22 | 4.74 | 5.18 | 5.86 | 6.30 |
| 2.HF (g) | 0.00 | 1.64 | 2.74 | 3.52 | 3.90 | 4.70 | 6.60 | 7.98 | 8.66 | 9.42 | 9.84 |
| 3. Control (g) | 0.00 | 0.50 | 1.00 | 1.40 | 2.10 | 2.27 | 3.27 | 3.50 | 3.77 | 4.65 | 5.10 |

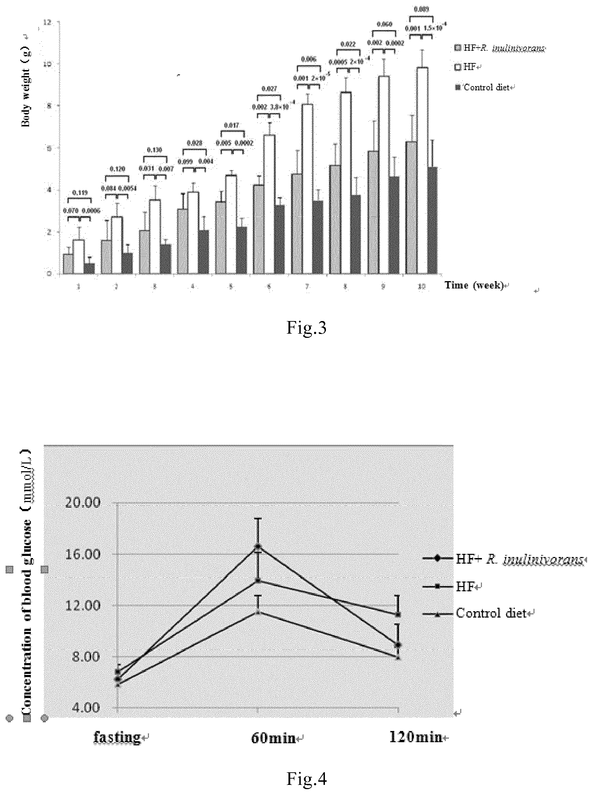

2. The P values of average increase of body weight during the 10 weeks treatment were shown in Table 2 and Fig. 3.

**Table 2**

| Grou p | Time (week) | | | | | | | | | |
|---|---|---|---|---|---|---|---|---|---|---|
| | 1 | 2 | 3 | 4 | 5 | 6 | 7 | 8 | 9 | 10 |
| PI-2 | 0.070 | 0.084 | 0.03 1 | 0.09 9 | 0.005 | 0.002 | 0.001 | 0.000 5 | 0.002 | 0.001 |
| P1-3 | 0.119 | 0.120 | 0.13 0 | 0.02 8 | 0.017 | 0.027 | 0.006 | 0.022 | 0.060 | 0.089 |
| P2-3 | 0.0006 | 0.0054 | 0.007 | 0.004 | 0.0002 | 0.00038 | 0.00002 | 0.0002 | 0.0002 | 0.00015 |

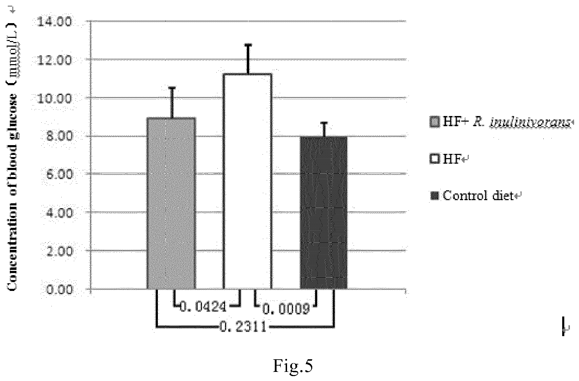

It was shown that the body weight of HFR group was significantly lower than HF group from Table 1, Table 2, Fig. 2 and Fig. 3. The results suggested that *R.inulinivorans* DSM 16841 could help with the mitigation of obesity in mice.
3. The blood glucose content of mice after 10 weeks was shown as Table 3, Table 4 and Fig. 4, Fig. 5.

**Table 3**

| Group | empty stomach (mmol/L) | 60min (mmol/L) | 120min (mmol/L) |
|---|---|---|---|
| 1. | 6.26 | 16.62 | 8.92 |
| 2. | 6.80 | 13.92 | 11.28 |
| 3. | 5.85 | 11.53 | 7.98 |

Table 3 and Fig. 4 indicated that the *R.inulinivorans* DSM 16841 could reduce the blood glucose of obese mice remarkably.

**Table 4**

| P Value | Empty stomach | 60 min | 120min |
|---|---|---|---|
| P1-2 | 0.1238 | 0.0868 | 0.0424 |
| P1-3 | 0.3511 | 0.0009 | 0.2311 |
| P2-3 | 0.0641 | 0.0495 | 0.0009 |

Table 4 and Fig. 5 indicated that the *R.inulinivorans* DSM 16841 could improve the blood glucose tolerance of obese mice remarkably.

### EXAMPLE 4

### Food Compositions Containing R. inulinivorans DSM 16841

The components of the food compositions are shown in Table 5.

**Table 5**

| Raw materials | percentage of content (%) |
|---|---|
| *R. inulinivorans* DSM 16841 | 0.5 |
| milk | 90.0 |
| sugar | 9.5 |

The components were mixed according to above mentioned ratio, stirred until mixed completely, and then preheated with high-pressure homogenization at 20Mpa. The compositions were sterilized for 5-10 minutes at 90°C, and inoculated when the temperature cooled to 40-43°C. The amount of inoculation was 1-100×10⁶cfu *R. inulinivorans* DSM 16841 /g.

### EXAMPLE 5

### Pharmaceutical Compositions Containing R. inulinivorans DSM 16841

The components of the pharmaceutical compositions are shown in Table 6.

**Table 6**

| Raw materials | percentage of content (%) |
|---|---|
| *R.inulinivorans* DSM 16841 | 1.0% |
| lactose | 2.0% |
| yeast extract | 2.0% |
| peptone | 1.0% |
| pure water | =-94.0% |

Lactose, yeast extract, peptone, and pure water were mixed according to above mentioned ratio, and preheated to 60-65°C with high-pressure homogenization at 20Mpa. The mixture was sterilized for 20-30 minutes at 90°C. *R. inulinivorans* DSM 16841(1-50×10⁶cfu/mL) was inoculated to the mixture when the temperature of the mixture was cooled to 36-38°C. After the resulting mixture fermented until pH turned to 6.0, it was centrifuged and freeze-dried to reach less than 3% moisture content, ie. preparing *R*. *inulinivorans* DSM 16841 bacteria freeze-dried material. 0.5g *R. inulinivorans* DSM 16841 freeze-dried material was then mixed with equal amount of malto dextrin, and encapsulated to form a pharmaceutical composition containing *R. inulinivorans* DSM 16841.

## Claims

1. A bacterial strain for use in the prevention and/or treatment of obesity, wherein the bacterial strain is selected from the *Roseburia* family.

2. The bacterial strain for use according to claim 1, the bacterial strain is *Roseburia inulinivorans.*

3. The bacterial strain for use according to claim 1, the bacterial strain is *Roseburia inulinivorans* DSM 16841.

4. A food composition comprising a pharmaceutically effective amount of a bacterial strain selected from the *Roseburia* family and an acceptable carrier for use in reducing blood glucose and/or body weight of subjects, wherein the bacterial strain is not *Roseburia hominis.*

5. The food composition for use according to claim 4, wherein forms of the food composition are selected from solid, dairy, solution, powder, and/or suspension.

6. A pharmaceutical composition comprising a pharmaceutically effective amount of a bacterial strain selected from the *Roseburia* family for use in reducing blood glucose and/or body weight of subjects, wherein the bacterial strain is not *Roseburia hominis.*

7. The pharmaceutical composition for use according to claim 6, wherein forms of the pharmaceutical composition are selected from granule, capsule, tablet, powder agent, oral liquid, suspension, and/or emulsion.

8. The bacterial strain according to claim 1, the food composition according to claim 4 or the pharmaceutical composition according to claim 6 for use in a method of reducing blood glucose and/or body weight comprising applying the bacterial strain according to claim 1, the food composition according to claim 4 or the pharmaceutical composition according to claim 6 to subjects in need thereof.

9. A bacterial strain selected from the *Roseburia* family for use in improving the tolerance of blood glucose.

## Patentansprüche

1. Ein Bakterienstamm zur Verwendung bei der Prävention und/oder Behandlung von Adipositas, wobei der Bakterienstamm ausgewählt ist aus der Familie *Roseburia.*

2. Der Bakterienstamm zur Verwendung gemäß Anspruch 1, der Bakterienstamm ist *Roseburia inulinivorans.*

3. Der Bakterienstamm zur Verwendung gemäß Anspruch 1, Der Bakterienstamm ist *Roseburia inulinivorans* DSM 16841.

4. Eine Nahrungsmittelzusammensetzung umfassend eine pharmazeutisch wirksame Menge von einem Bakterienstamm ausgewählt aus der Familie *Roseburia* und einen verträglichen Träger zur Verwendung bei dem Reduzieren von Blutglukose und/oder Körpergewicht eines Subjekts, wobei der Bakterienstamm nicht *Roseburia hominis* ist.

5. Die Nahrungsmittelzusammensetzung zur Verwendung gemäß Anspruch 4, wobei die Arten der Nahrungsmittelzusammensetzung ausgewählt sind aus Feststoff, Milcherzeugnis, Lösung, Puder und/oder Suspension.

6. Eine pharmazeutische Zusammensetzung umfassend eine pharmazeutisch wirksame Menge von einem Bakterienstamm ausgewählt aus der Familie *Roseburia* zur Verwendung bei dem Reduzieren von Blutglukose und/oder Körpergewicht eines Subjekts, wobei der Bakterienstamm nicht *Roseburia hominis* ist.

7. Die pharmazeutische Zusammensetzung zur Verwendung gemäß Anspruch 6, wobei die Arten der pharmazeutischen Zusammensetzung ausgewählt sind aus Granulat, Kapsel, Tablette, Pudermittel, orale Flüssigkeit, Suspension und/oder Emulsion.

8. Der Bakterienstamm gemäß Anspruch 1, die Nahrungsmittelzusammensetzung gemäß Anspruch 4 oder die pharmazeutische Zusammensetzung gemäß Anspruch 6 zur Verwendung in einem Verfahren zum Reduzieren von Blutglukose und/oder Körpergewicht umfassend das Anwenden des Bakterienstamms gemäß Anspruch 1, der Nahrungsmittelzusammensetzung gemäß Anspruch 4 oder der pharmazeutischen Zusammensetzung gemäß Anspruch 6 bei Subjekten, welche es benötigt.

9. Ein Bakterienstamm ausgewählt aus der Familie *Roseburia* zur Verwendung bei der Verbesserung der Toleranz von Blutglukose.

## Revendications

1. Souche bactérienne destinée à être utilisée dans la prévention et/ou le traitement de l'obésité, sachant que la souche bactérienne est sélectionnée dans la famille Roseburia.

2. La souche bactérienne destinée à être utilisée selon la revendication 1, la souche bactérienne étant Roseburia inulinivorans.

3. La souche bactérienne destinée à être utilisée selon la revendication 1, la souche bactérienne étant Roseburia inulinivorans DSM 16841.

4. Composition alimentaire comprenant une quantité pharmaceutiquement efficace d'une souche bactérienne sélectionnée dans la famille Roseburia et un support acceptable, destinée à être utilisée dans la réduction du glucose sanguin et/ou du poids corporel de sujets, sachant que la souche bactérienne n'est pas Roseburia hominis.

5. La composition alimentaire destinée à être utilisée selon la revendication 4, sachant que des formes de la composition alimentaire sont sélectionnées parmi un solide, un produit laitier, une solution, une poudre, et/ou une suspension.

6. Composition pharmaceutique comprenant une quantité pharmaceutiquement efficace d'une souche bactérienne sélectionnée dans la famille Roseburia, destinée à être utilisée dans la réduction du glucose sanguin et/ou du poids corporel de sujets, sachant que la souche bactérienne n'est pas Roseburia hominis.

7. La composition pharmaceutique destinée à être utilisée selon la revendication 6, sachant que des formes de la composition pharmaceutique sont sélectionnées parmi un granule, une capsule, un comprimé, un agent en poudre, un liquide oral, une suspension, et/ou une émulsion.

8. La souche bactérienne selon la revendication 1, la composition alimentaire selon la revendication 4 ou la composition pharmaceutique selon la revendication 6, destinées à être utilisées dans un procédé de réduction du glucose sanguin et/ou du poids corporel, comprenant l'application de la souche bactérienne selon la revendication 1, de la composition alimentaire selon la revendication 4 ou de la composition pharmaceutique selon la revendication 6 à des sujets qui en ont besoin.

9. Souche bactérienne sélectionnée dans la famille Roseburia, destinée à être utilisée dans l'amélioration de la tolérance de glucose sanguin.
